(19) Europäisches Patentamt
European Patent Office
Office européen des brevets

(11) Publication number : 0 493 030 A1

(12) # EUROPEAN PATENT APPLICATION

(21) Application number : 91311885.7

(51) Int. Cl.[5] : C07C 25/13, C07C 17/22

(22) Date of filing : 20.12.91

(30) Priority : 21.12.90 GB 9027945

(43) Date of publication of application :
01.07.92 Bulletin 92/27

(84) Designated Contracting States :
AT BE CH DE DK ES FR GB GR IT LI LU NL SE

(71) Applicant : RHONE-POULENC CHEMICALS LIMITED
Oak House, Reeds Crescent
Watford, Herts WD1 1QM (GB)

(72) Inventor : Russell, James John
5 Wellington Walk
Westbury on Trym, Bristol BS10 5ET (GB)

(74) Representative : Collier, Jeremy Austin Grey
J.A.Kemp & Co., 14 South Square, Gray's Inn
London WC1R 5LX (GB)

(54) **Preparation of difluorobenzenes.**

(57) Difluorobenzenes are prepared by reacting a fluoronitrobenzene of the formula :

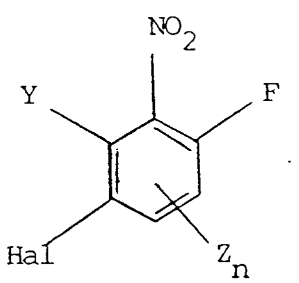

in which Hal represents fluorine, chlorine or bromine, Y represents hydrogen or methyl, Z represents hydrogen or a weakly electrodonating group on a non-electrodonating group and n is 1 to 2, with a source of fluoride ion to replace the nitro group by fluorine. The products are useful intermediates in the preparation of drugs based on a quinoline structure.

EP 0 493 030 A1

This invention relates to the synthesis of difluorobenzenes which are useful chemical intermediates for drugs based on a quinoline structure, which have anti-bacterial properties.

British Patent No. 830,832 describes substituted quinoline derivatives of generally N-alkyl quinolone structure with the heterocyclic ring substituted with alkyl and carboxyl groups. More recently quinolone based drugs have been devised wherein the carbocyclic ring in the quinolone nucleus is substituted with halogen atoms, especially fluorine and chlorine atoms. Such compounds are disclosed, inter alia, in European Patent specifications 000,203 and 0,303,291, Belgian Patent 863,429, French Patents 2,210,413 and 2,257,292 and US Patent 4,730,000.

A valuable precursor for making some of these halogen substituted N-alkyl, N-cycloalkyl, N-aryl or N-aralkyl quinoline derivatives is the compound 1-chloro-3,4-difluorobenzene. This can easily be converted to the key intermediate a 2,4-halogeno-5-fluorobenzoic acid such as 2-chloro-4,5-difluorobenzoic acid by acetylation and subsequent oxidation of the ensuing acetophenone and this benzoic acid can thence be converted to a suitable quinolone structure - e.g. by the methods disclosed in European Patent specification 0,303,291.

However, 1-chloro-3,4-difluorobenzene has proved to be extremely difficult to synthesise on a commercial scale. Methods of preparing 1-chloro-3,4-difluorobenzene have included the diazotisation of 3,4-difluoroaniline in aqueous hydrochloric acid with aqueous sodium nitrite followed by a reaction with cuprous chloride/aqueous hydrochloric acid (see JACS, 1959, 81, 94-101). While this reaction can be carried out on a large scale without difficulty, the starting material, 3,4-difluoroaniline, cannot be prepared economically on the tonnage scale by known commercial processes and it is, therefore, expensive and difficult to acquire.

It has also been proposed to obtain 1-chloro-3,4-difluorobenzene by reacting chloroaromatic compounds with silver difluoride (see J.Org.Chem., 1980, 45 (18), 3597-3603) but silver difluoride is an expensive material which is difficult to handle. Added to this the process is complex and consists of several stages such that it is not economically viable.

Again the target compound can be prepared by the pyrolysis of vinyl fluorocyclobutanes (see US Patent No. 4754084) but the starting materials are expensive. Added to this selectivity to the desired product is low.

1,2,4-trichlorobenzene has also been used as starting material since it is a readily available material. In one process it is reacted with potassium fluoride in dimethylsulphone (see J.Fluorine Chem., 1972-73, 2 (1), 19-26) but the yield is only 8% so that the process is not commercially viable. The product is mixed with isomers which make it difficult to isolate the desired product and the process has to be carried out in expensive pressure equipment which, since corrosion from chloride ion can be expected, has to be made from expensive materials. 1,2,4-trichlorobenzene has also been reacted with a nitrosyl fluoride/hydrogen fluoride complex mixture over cobalt fluoride at 420°C (see French patent No. 1385681). These reagents are toxic and expensive and the yield of the desired product is poor.

Accordingly, it can be seen that none of the processes described for the production of 1-chloro-3,4-difluorobenzene is suitable for use on a commercial scale. There is, therefore, a need for an economically viable route for obtaining this compound and related difluorobenzenes in order to meet the growing demand for specialised fluorinated quinolone antibacterial drugs, in particular.

According to the present invention there is provided a process for the preparation of a difluorobenzene which comprises reacting a fluoronitrobenzene of the formula:

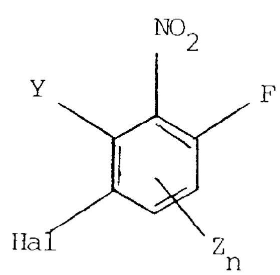

in which Hal represents fluorine, chlorine or bromine, preferably chlorine, Y represents hydrogen or methyl, preferably hydrogen, Z represents hydrogen or a weakly electrodonating group or a non-electrodonating group, preferably in the 4-position with respect to the nitro group, such as a linear or branched alkyl of 1 to 10, especially 1 to 4, carbon atoms, including cycloalkylalkyl or aralkyl, e.g. cyclohexylmethyl or benzyl, nitrile, fluoroalkyl or polychloroalkyl, e.g. $-CCl_3$, $-CHCl_2$, $-CH_2Cl$, -COCl or -COF, -OR, -COR or -COOR where R = $C_{1-10}$ alkyl or aryl (e.g. phenyl), aldehydo, dialkylamido, e.g. $-CONMe_2$, halo-sulphonyl, e.g. $-SO_2Cl$ or $-SO_2F$, or alkyl or fluoroalkyl-sulphonyloxy e.g. $-OSO_2R_f$ where $R_f$ = perfluoroalkyl, and n is 1 or 2, such that when n is 2 each Z can be

the same or different, with a source of fluoride ion, and preferably in an aprotic solvent. Naturally the substituent which Z represents should not interfere with the reaction between the nitro group and the fluoride source.

The general process, in which the nitro group on an aromatic ring is subjected to attack by fluoride ion in an aprotic solvent, resulting in the formation of the relevant fluoroaromatic compound, was first published by G.C. Finger and C.W. Kruse, J.Amer.Chem.Soc., 1956, 78, 6034-6037.

Preferred starting materials are those in which Hal is chlorine, Y is hydrogen, n is 1 and Z is hydrogen or a substituent in the 4-position selected from methyl, possibly substituted by 2 or, preferably, 1 halogen, advantageously fluorine.

The preferred starting material is 5-chloro-2-fluoronitrobenzene. 5-Chloro-2-fluoronitrobenzene may be introduced into the reaction mixture either directly, or indirectly, by adding 2,5-dichloronitrobenzene, or less preferably 2-bromo-5-chloronitrobenzene, to a source of fluoride ion in an aprotic solvent, preferably a dipolar solvent if a phase transfer catalyst is not present. The 5-chloro-2-fluoro-nitrobenzene is then formed in situ as an intermediate, before the next stage of fluorodenitration to 1-chloro-3,4-difluorobenzene takes place.

Other valuable starting materials are 2-fluoro-5-bromonitrobenzene and 2,5-difluoro-nitrobenzene. The former can be produced in situ by adding 2,5-dibromo-nitrobenzene to a source of fluoride ion in an aprotic solvent in the same way as that described above for the corresponding 2-fluoro-5-chloro compound.

Suitable sources of fluoride are well known to those skilled in the art. Preferred fluorides include alkali metal fluorides such as sodium, potassium and caesium fluorides, or an admixture of any two, or all three of these. While fluorides of the higher molecular weight alkali metals generally give better results, for economic reasons the use of potassium fluoride is especially preferred. Quaternary ammonium or phosphonium fluorides, e.g. a tetraalkylammonium fluoride, preferably tetramethylammonium fluoride, can also be used.

While the reaction can be carried out in the absence of a solvent, it is preferably effected in an aprotic solvent, e.g. dimethyl sulphoxide, dimethyl formamide, dimethyl acetamide, benzonitrile, dimethyl sulphone, sulpholane, N-methyl pyrrolidone (NMP), 1,3-dimethyl-2-imidazolidinone (DMI), 2-methyl-naphthalene, tetralin and 1-chloronaphthalene. The amount of solvent used is not critical.

The reaction requires substantially anhydrous conditions, i.e. the water content of the reaction mixture should be below about 1% by weight. When, as is preferred, an acyl halide nitrite ion trap is used as described below, the reaction mixture is essentially self drying.

Any solvent used must, of course, be compatible with the other components of the reaction mixture. For example, when an acyl halide nitrite ion trap is used the solvent cannot be an amide or dimethyl sulphoxide. Since the reaction is conveniently effected at ambient pressure and high reaction temperatures (over 200°C) are often preferred to ensure a satisfactory rate of reaction, it is preferred in these circumstances to use a solvent which boils at high temperature under ambient pressure and remains stable at the chosen reaction temperature. Choice of a suitable solvent meeting these requirements is within the ordinary skill of the art.

The reaction is conveniently carried out at elevated temperature. The general temperature range is from 20° to 350°C, depending on the solvent being used. More specifically, a temperature range of 50° to 275°C is conveniently used. The preferred temperature range is 130° to 275°C.

A nitrite ion trap is preferably present during the reaction. (See R.L. Markezich, O.S. Zamek, P.E. Donahue, and F.J. Williams, J.Org.Chem., 1977, 42, 3435-3436, who studied the effect of the nitrite ion during the fluorodenitration of 4-nitrophthalic anhydride). Examples of nitrite ion traps include higher boiling acyl halides, e.g. chlorides or fluorides, such as benzene sulphonyl chloride, phthaloyl dichloride, phthaloyl difluoride, benzoyl chloride, pyromellitic acid tetrachloride and naphthalene-1,8-dicarbonyl dichloride. It is believed that, under the reaction conditions, an acyl chloride reacts with the fluoride to produce the corresponding acyl fluoride, The trap should generally be used in an amount from 1 to 5 moles per mole of the expected desired product although there is no real upper limit. To be fully effective it should be present in excess.

In general the source of fluoride ion should be present in excess but there is no real upper limit; since it will react with some nitrite traps the minimum requirement is the stoichiometric amount necessary to react plus a molar amount equivalent to that of the nitrobenzene starting material. Typically 1 to 5 moles of fluoride are used in relation to the minimum molar amount.

Preferably the reaction is carried out in the presence of a phase transfer catalyst (see Markezich et al loc.cit), especially if the solvent used is not dipolar. Examples of phase transfer catalysts include quaternary ammonium and phosphonium salts in which the substituent radicals may be alkyl, aralkyl, or aryl or a combination thereof, and the anion is typically fluoride, chloride or bromide, e.g. tetramethyl ammonium fluoride or chloride, tetrabutylammonium bromide, cetyl trimethyl ammonium bromide, tricapryl methyl ammonium chloride (ALIQUAT 336(R)), tetrabutylphosphonium bromide, tetraphenyl phosphonium bromide, tetraphenyl phosphonium chloride, benzyl trimethyl ammonium chloride, and benzyl triphenyl phosphonium bromide; crown ethers, e.g. 18-crown-6; polyethylene glycols; and N-alkyl-4-N′,N′-dialkylamino-pyridinium salts, for example N-neopentyl-4-(N′,N′-dimethylamino)-pyridinium chloride and N-(2-ethylhexyl)-4-(N′,N′-dimethylamino)-pyridinium chloride. Combinations of phase transfer catalyst may be used and it will be appreciated that the source of fluoride ion may itself be a phase transfer catalyst if it includes a suitable cation. The preferred phase transfer catalyst is tetraphenyl phosphonium chloride or bromide.

The reaction may be run at atmospheric pressure in, say, glass apparatus, but may also be run at elevated pressures (e.g. up to 2000 kPa) in a suitable autoclave made of a suitable material such as Hastelloy C(R).

While the reaction may be conveniently run by combining all reagents (e.g. solvent, fluoride ion source, organic nitro compound, phase transfer catalyst, and nitrite trap) together in suitable reaction equipment, and bringing the stirred mixture to the desired temperature for the specified time before working up, other methods of carrying out the reaction may be employed. For example, the fluoride ion source, phase transfer agent, and solvent may be combined by stirring, and brought to the reaction temperature, and then a mixture of the organic nitro compound and nitrite trap may be progressively added to the reaction mixture. Again, aliquots of phase transfer agent may be added during the reaction to maintain an adequate concentration. Such variations in operating procedure may add significantly to the efficiency of the process, and are to be regarded as being within the scope of this specification.

The product is conveniently isolated by distillation out of the reaction mixture and when the product boils at a temperature below the reaction temperature it is preferably distilled out as it is formed.

The following Examples further illustrates the present invention.

## EXAMPLE 1

### Preparation of 1-chloro-3,4-difluorobenzene from 5-chloro-2-fluoronitrobenzene by fluorodenitration

Into a 250 ml three necked round bottom flask fitted with a thermocouple, mechanical stirrer and swan neck adaptor fitted with a septum inlet and a reflux condenser was added potassium fluoride (28.0 g., 0.482 mole) and sulpholane (125.5 g.). The system was purged with nitrogen and 5-chloro-2-fluoronitrobenzene (17.1 g. of purity 98.6% containing 0.096 mole), o-phthaloyl dichloride (30.3 g. of purity 95%, containing 0.142 mole) were added. The mixture was heated under nitrogen at 210°C for 3 hours and then at 220°C for 5 hours. At the end of this period the mixture was allowed to cool. Methylene chloride (100 ml) was added and the resulting slurry was filtered. The filtrate was concentrated under atmospheric pressure to yield 177.2 g. of the crude product. Composition by glc: 1-chloro-3,4-difluoro-benzene: 5.1%, 5-chloro-2-fluoronitrobenzene: 1.7%, sulpholane: 67.2%, and phthalic anhydride: 11.2%. The reaction yield to 1-chloro-3,4-difluorobenzene was therefore 64%, the conversion of 5-chloro-2-fluoronitrobenzene to product was 82%, and the selectivity to the desired product, 1-chloro-3,4-difluorobenzene, was 78%. Fractional distillation or steam distillation of the crude product yielded the pure desired product (b.pt. 127°C at atmospheric pressure) in good recovery.

## EXAMPLES 2-19

Examples 2-19 were carried out in a similar manner to Example 1, but differences in reagents and conditions used are specified in the following Table, together with the data from Example 1.

From this table it can be seen that a polar aprotic, preferably high boiling, solvent such as sulpholane is a good medium for the double exchange (fluorodechlorination and fluorodenitration) and between about 200°C and about 205°C.

It can also be seen that for fluorodenitration a non-polar solvent gives very good results in the same temperature range, especially with a phosphonium derivative as a phase transfer catalyst. It has surprisingly been found that the yields obtained using the process according to the invention where a double exchange is performed are high, generally higher than 40%, in some cases higher than 50% and even in some cases higher than 60%. Generally such yields are obtained using preferred conditions.

The yield is also high in the monoexchange process, i.e. fluorodenitration. Yields may be higher than 50%, in some cases higher than 60% and even, in some cases, higher than 70%. Again such yields are generally the result of using preferred process conditions.

| Example No. | Starting Material (0.1 mole) | Fluoride Source | | Solvent (aprotic) | | Reaction Conditions | | Phase Transfer Catalyst | | Nitrite ion Trap | | Reaction Results | | |
|---|---|---|---|---|---|---|---|---|---|---|---|---|---|---|
| | | type | moles | type | moles | Temp. (°C) | Time (hr) | type | moles | type | moles | Con(1) | Yld(2) | (3) |
| 1 | 5C2FNB(4) | KF | 0.48 | Sulpholane | 1.05 | (210<br>(then<br>(220 | 3.0<br><br>5.0 | None | | PDC(6) | 0.14 | 82 | 64 | 78 |
| 2 | " | " | 0.5 | " | 1.1 | 230 | 10.0 | TPPBr(5) | - | " | 0.15 | 62 | 43 | 69 |
| 3 | " | " | 0.5 | " | 1.1 | 220 | 10.0 | " | - | " | 0.15 | 49 | 28 | 57 |
| 4 | " | " | 0.4 | 1-Chloronaphthalene | 0.4 | 220 | 20.0 | " | 0.01 | PDC | 0.1 | 46 | 33 | 72 |
| 5 | " | " | 0.4 | " | 0.4 | 230 | 0.5 | " | 0.003 | " | 0.1 | 79 | 66 | 84 |
| 6 | " | " | 0.4 | 2-methylnaphthalene | 0.4 | 220-230 | 0.5 | " | 0.005 | " | 0.1 | 41 | 22 | 54 |
| 7 | " | " | 0.4 | " | 0.4 | 230 | 0.5 | " | 0.01 | " | 0.1 | 40 | 10 | 25 |
| 8 | " | " | 0.4 | 1-Chloronaphthalene | 0.4 | 230 | 0.5 | " + Quinoline | 0.003+0.10 | " | 0.1 | 56 | 49 | 88 |
| 9 | " | " | 0.4 | " | 0.4 | 240 | 0.5 | " | 0.0012 | " | 0.1 | 55 | 47 | 86 |
| 10 | " | " | 0.4 | " | 0.4 | 250 | 0.5 | " | 0.0012 | " | 0.1 | 64 | 32 | 50 |
| 11 | " | " | 0.4 | " | 0.4 | 230 | 0.5 | TPPCl(7) | 0.010 | " | 0.1 | 100 | 82 | 82 |
| 12 | " | (KF<br>(CsF | 0.4<br>0.007 | " | 0.4 | 230 | 1.0 | TPPBr | 0.003 | " | 0.1 | 91 | 78 | 86 |
| 13 | " | KF | 0.4 | Tetralin | 0.4 | 206-214 | 12.0 | " | 0.003 | " | 0.1 | 100 | 17 | 17 |
| 14 | " | " | 0.4 | No solvent | | 230 | 0.5 | " | 0.003 | " | 0.1 | 89 | 62 | 70 |
| 15 | " | $Me_4NF$ | 0.24 | Dimethyl sulphoxide | 1.7 | 60-80 | 2.25 | none | | none | | 96 | 21 | 22 |
| 16 | " | (KF<br>(CsF | 0.2<br>0.2 | Sulpholane | 0.4 | 140-170 | 4.0 | $Me_4NCl$ | 0.1 | PDC | 0.1 | 30 | 15 | 50 |
| 17 | 2,5DCNB(8) | KF | 0.5 | Sulpholane | 0.4 | 230 | 2.0 | TPPBr | 0.005 | " | 0.1 | 90 | 64 | 71 |
| 18 | 2,5DFNB(9) | " | 0.4 | 1-Chloronaphthalene | 0.4 | 230 | 2.0 | " | 0.003 | " | 0.1 | 52(10) | 33(10) | 64(10) |
| 19 | 5Br2FNB(11) | " | 0.4 | " | 0.4 | 230 | 0.5 | " | 0.003 | " | 0.1 | 83(12) | 78(12) | 93(12) |

(1) Conversion )
(2) Yield } to 1-chloro-3,4-difluorobenzene
(3) Selectivity )
(4) 5-Chloro-2-fluoronitrobenzene
(5) Tetraphenylphosphonium bromide
(6) O-phthaloyldichloride
(7) Tetraphenylphosphonium chloride
(8) 2,5-Dichloronitrobenzene
(9) 2,5-Difluoronitrobenzene (0.1 mole)
(10) to 1,2,4,-trifluorobenzene
(11) 5-Bromo-2-fluoronitrobenzene (0.076 mole)
(12) to 1-bromo-3,4-difluorobenzene

## Claims

1. A process for the preparation of a difluorobenzene which comprises reacting a fluoronitrobenzene of the formula:

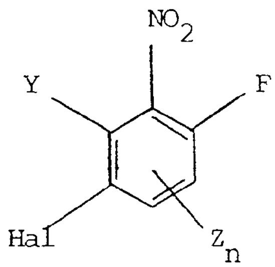

in which Hal represents fluorine, chlorine or bromine, Y represents hydrogen or methyl, Z represents hydrogen or a weakly electrodonating group or a non-electrodonating group and n is 1 or 2 with a source of fluoride ion.

2. A process according to claim 1 in which Hal represents chlorine, Y represents hydrogen, n is 1, and Z represents hydrogen or 4-methyl.

3. A process according to claim 1 in which the starting material is 5-chloro-2-fluoronitrobenzene.

4. A process according to claim 3 in which the 5-chloro-2-fluoro-nitrobenzene is produced in situ by the reaction of 2,5-dichloronitrobenzene with a source of fluoride ion.

5. A process according to any one of claims 1 to 4 in which the source of fluoride ion is potassium fluoride, or tetramethylammonium fluoride.

6. A process according to any one of claims 1 to 5 in which the reaction is effected in an aprotic solvent.

7. A process according to claim 6 in which the aprotic solvent is sulpholane, 1-chloronaphthalene, 2-methylnaphthalene, tetralin, or dimethylsulphoxide.

8. A process according to any one of claims 1 to 7 which is carried out at a temperature of 50° to 275°C.

9. A process according to any one of claims 1 to 8 which is carried out in the presence of a phase transfer catalyst.

10. A process according to claim 9 in which the phase transfer catalyst is tetramethylammonium chloride, or tetraphenylphosphonium chloride or bromide.

11. A process according to any one of claims 1 to 10 which is carried out in the presence of a nitrite ion trap.

12. A process according to claim 11 in which the said nitrite ion trap is o-phthaloyldichloride.

European Patent Office

# EUROPEAN SEARCH REPORT

Application Number

EP 91 31 1885

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | JOURNAL OF THE AMERICAN CHEMICAL SOCIETY. vol. 78, 5 December 1956, EASTON, PA US pages 6034 - 6037; G. C. FINGER ET AL: 'Aromatic Fluorine Compounds. VII Replacement of Aromatic -Cl and -NO2 Groups by -F' * page 6035, left column, paragraph 1 * * page 6036, right column * | 1,5,7 | C07C25/13<br>C07C17/22 |
| | ----- | | |
| | | | TECHNICAL FIELDS SEARCHED (Int. Cl.5 ) |
| | | | C07C |

The present search report has been drawn up for all claims

| Place of search | Date of completion of the search | Examiner |
|---|---|---|
| THE HAGUE | 23 MARCH 1992 | J. VAN GEYT |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)